# EUROPÄISCHE PATENTSCHRIFT

(11) **EP 2 274 005 B1**
(45) Veröffentlichungstag und Bekanntmachung des Hinweises auf die Patenterteilung: **29.05.2013**
(21) Anmeldenummer: 09730771.4
(22) Anmeldetag: 07.04.2009
(51) Int. Cl.: A61K 38/48, A61P 35/00

(54) **BEHANDLUNG VON TUMORERKRANKUNGEN DURCH ANGIOTENSIN KONVERTIERENDES ENZYM 2 (ACE2)**
TREATMENT OF TUMORS BY ANGIOTENSIN CONVERTING ENZYME 2 (ACE2)
TRAITEMENT DE MALADIES TUMORALES PAR L'ENZYME DE CONVERSION DE L'ANGIOTENSINE 2 (ACE2)

(30) Priorität: 09.04.2008 AT 5662008
(43) Veröffentlichungstag der Anmeldung: 19.01.2011
(60) Teilanmeldung: 13162709.3
(73) Patentinhaber: Apeiron Biologics AG, 1030 Wien (AT)
(72) Erfinder: JANZEK-HAWLAT, Evelyne, A-1230 Wien (AT); LOIBNER, Hans, A-1230 Wien (AT); SCHUSTER, Manfred, A-2191 Schrick (AT); PEBALL, Bernhard, A-1070 Wien (AT)
(74) Vertreter: Sonn & Partner Patentanwälte
(86) Internationale Anmeldenummer: PCT/AT2009/000136
(87) Internationale Veröffentlichungsnummer: WO 2009/124330

(56) Entgegenhaltungen:
- WO-A-2004/000367
- DATABASE BIOSIS [Online] BIOSCIENCES INFORMATION SERVICE, PHILADELPHIA, PA, US; April 2008 (2008-04), LOIBNER HANS ET AL: "Development of recombinant human soluble angiotensin converting enzyme 2 (rkACE2) for cancer therapy" XP002549481 Database accession no. PREV200800488340 & PROCEEDINGS OF THE AMERICAN ASSOCIATION FOR CANCER RESEARCH ANNUAL MEETING, Bd. 49, April 2008 (2008-04), Seite 969, 99TH ANNUAL MEETING OF THE AMERICAN-ASSOCIATION-FOR-CANCER-RESEARCH; SAN DIEGO, CA, USA; APRIL 12 -16, 2008 ISSN: 0197-016X

## Beschreibung

Die Erfindung betrifft die Behandlung von Tumorerkrankungen.

Nach Angaben der amerikanischen Krebsgesellschaft (American Cancer Society) werden pro Jahr in den Industrieländern 5,4 Millionen Menschen neu an Krebs erkranken, in den weniger entwickelten Ländern insgesamt 6,7 Millionen, was die Zahl der neuen Krebsfälle auf über zwölf Millionen pro Jahr klettern lässt. Insgesamt 7,7 Millionen Menschen weltweit sterben derzeit jährlich an einem Tumorleiden. Die drei häufigsten Krebserkrankungen in den Industrieländern sind Prostata-, Lungen- und Darmtumoren, wobei Frauen am häufigsten unter Brust-, Darm- und Lungenkrebs leiden. Da Infektionskrankheiten beherrschbar geworden sind und die Menschen immer älter werden, nehmen Krebserkrankungen in den Todesstatistiken der Industrieländer einen immer höheren Stellenwert ein.

In den Entwicklungsländern verschiebt sich das Bild: Hier sind Lungen-, Magen- und Leberkrebs die häufigsten Krebserkrankungen der Männer. Frauen leiden am häufigsten unter Brust-, Gebärmutterhals- und Magenkrebs. Magen- und Gebärmutterhalskrebs sind dabei in der Regel die Folge einer Infektion.

Insgesamt sind 15 Prozent aller Krebsfälle auf Krankheitserreger zurückzuführen, von denen Menschen, die in Entwicklungsländern leben, häufiger betroffen sind. Bei ihnen sind 26 Prozent der Tumoren Folgen von Infektionen, in Industrieländern nur sechs Prozent.

In Österreich, wie auch in anderen Industriestaaten, sind Krebserkrankungen die zweithäufigste Todesursache, nur übertroffen von den Erkrankungen des Herz-Kreislauf-Systems. In Deutschland erkranken etwa 395.000 Menschen jährlich an Krebs, davon rund 195.000 Frauen und 200.000 Männer. Die meisten Fälle treten im Alter von über 60 Jahren auf. Die unter 60-jährigen machen mit etwa 107.000 Fällen nur rund ein Viertel der Krebs-Neuerkrankungen aus.

Viele Tumortherapien, wie zB. Strahlungstherapie, Chemotherapie oder operative Tumorentfernungen, sind seit Jahren etabliert und werden ständig verfeinert und verbessert. Neue Therapien umfassen Immuntherapien, Therapien, die auf Blutgefäßneubildung oder aber auf spezifische Marker von Tumorzellen abzielen, zB. unter Verwendung von monoklonalen Antikörpern.

Trotz der verbesserten Therapiemöglichkeiten für viele Tumore, die in den letzten 30 Jahren entwickelt wurden, ist der Kampf gegen den Krebs nicht gewonnen, wie man noch Anfang der siebziger Jahre in naher Zukunft erwartete.

Selbst in den Industrieländern liegt die derzeitige Heilungsrate bei Krebs bei ca. 30 bis 65 Prozent (USA: 65 Prozent), wenn man alle verschiedenen Krebserkrankungen bei beiden Geschlechtern zusammenfasst. Im Einzelfall sind die Heilungschancen jedoch höchst unterschiedlich: Oft stehen die Chancen gut, solange die Krebserkrankung örtlich begrenzt bleibt; wenn sich der Tumor bereits in mehreren Organen des Körpers ausgebreitet hat, sind die Chancen wesentlich geringer. Generell ist die frühe Erkennung einer Krebserkrankung äußerst wichtig. Auch können Patienten unterschiedlich auf eine Chemotherapie ansprechen, bei manchen Patienten sind gewisse Wirkstoffklassen völlig oder fast völlig unwirksam.

Sowohl die US 6,194,556 und die US 7,482,171 (beide durch Bezugnahme hierin eingeschlossen) beschreiben ACE2 Nukleinsäure- und Aminosäure-Sequenzen, funktionelle Varianten sowie Assays, um die ACE2-Aktivität zu bestimmen.

Zhou et al. (Tohoku J. Exp. Med. 217 (2009): 123-131; durch Bezugnahme hierin eingeschlossen) beschreiben Änderungen der Ang II-Konzentration und ACE2-Expressionswerte in Adenokarzinom-Gewebe des Pankreas (Pancreatic Ductal Adenocarcinoma - PDAC). Es wurde gefunden, dass Ang II in PDAC-Zelllinien akkumuliert und die Expression vom ACE2-Protein herunterreguliert. Es wurde darin darauf hingewiesen, dass das Verhältnis von ACE/ACE2 von besonderer Bedeutung ist und ein Ungleichgewicht darin zu diversen Krankheiten führen kann und bei der Pathogenese des PDAC involviert ist. Daher wurde ACE2 als molekulares Target zur Behandlung von PDAC vorgeschlagen.

Ein besonderes Problem bei vielen Behandlungsmethoden sind die schweren Nebenwirkungen. Oft sterben Krebspatienten nicht aufgrund der Tumorerkrankung, sondern aufgrund der zur Tumorbekämpfung eingesetzten Mittel und Methoden.

Es besteht daher noch immer ein großer Bedarf an innovativen Verbesserungen der Tumortherapie, insbesondere an Therapien, die auf Basis von körpereigenen Mitteln erfolgt und keine oder nur geringe Nebenwirkungen haben. Es ist daher Aufgabe der vorliegenden Erfindung, Therapien für Tumorerkrankungen zur Verfügung zu stellen, die effizient sind und keine oder zumindest keine schweren Nebenwirkungen aufweisen.

Demgemäß betrifft die vorliegende Erfindung die Verwendung eines Polypeptids mit Angiotensin konvertierender Enzym 2 (angiotensin converting enzyme 2; ACE2)-Aktivität zur Herstellung eines Medikaments zur Behandlung von Tumorerkrankungen, ausgenommen Lungenkrebs.

Gemäß der vorliegenden Erfindung werden Tumorerkrankungen mit ACE2-Aktivität behandelt. Dabei stellte sich heraus, dass die ACE2-Aktivität in der Lage ist, das Tumorzell-Wachstum effizient zu unterbinden, ohne dass dabei Nebenwirkungen auf Basis dieser Aktivität befürchtet werden müssen.

Über Jahrzehnte wurden dem Renin Angiotensin System (RAS) vorwiegend Funktionen der Homeostase und vor allem der Blutdruckregulierung zugeschrieben. Während der letzten Jahre wurden jedoch weitere Eigenschaften des RAS erkannt und dessen Aktionsradius um essentielle Funktionen wie die der Zellproliferation, der Angiogenese, der Inflammation und des pathologischen Gewebeumbaus erweitert. Ein Schlüsselpeptid des "aktivierten" RAS stellt hierbei Angiotensin II (Ang II) dar, welches als positiver Regulator des RAS vasokonstriktive, hypertensive, proinflammatorische, proliferative und pro-angiogenetische Eigenschaften aufweist. Ferner trägt Ang II zur Entstehung reaktiver Superoxide bei. All diese Eigenschaften begünstigen die zelluläre Entartung und tragen zur Entstehung maligner Tochterzellen bei. So bedient sich die entartete Zelle der Vaskularisierung, um zu einem Primärtumor heranzuwachsen. Es zeigte sich, dass Ang II in diversen soliden Tumorerkrankungen in erhöhten Mengen vorhanden ist.

Das maßgeblich für die Produktion für Ang II verantwortliche Enzym ist das "Angiotensin-convertierende Enzym" (ACE), welches das Dekapeptid Angiotensin I zu Ang II umwandelt. ACE nimmt, als ein Teil einer Blutdruck regulierenden Kaskade (wiederum des RAS), insbesondere in der Blutdruck-Regulierung eine zentrale Stellung ein. Eine hohe ACE-Aktivität steigert den Blutgefäßtonus und somit den Blutdruck. Die Hemmung von ACE ist daher ein erfolgreicher Therapieansatz zur Behandlung des Bluthochdruckes (Hypertonie). ACE-Hemmer, also Hemmstoffe (Inhibitoren) von ACE, wie Captopril, Enalapril, Lisinopril und Ramipril, zählen zu den umsatzstärksten Arzneistoffen überhaupt. ACE-Hemmer wurden daher schon seit längerem auf Basis der obigen Erwägungen auch im Bereich der Tumortherapie vorgeschlagen und verwendet.

In diesem Zusammenhang konnte auch gezeigt werden, dass neben einer erhöhten Konzentration von Ang II auch dessen Rezeptoren AT1 und AT2 (AT1R und AT2R) in vielen soliden Tumorerkrankungen signifikant überexprimiert werden. Ang II trägt hierbei wesentlich zur Tumor-neovaskularisierung und fortan zur Tumorinvasion bei. Es fördert weiters die Mitogen Aktivierte Protein (MAP)-Kinasen-Phosphorylierung sowie die VEGF-Sekretion. Beide Mechanismen sind als relevant bei der Entstehung und Versorgung solider Tumoren erkannt worden. Klinisch korreliert eine erhöhte AT1R-Expression mit erhöhten VEGF-Titern und einer deutlich verkürzten Überlebensprognose.

Daher wurden im Anschluss an die ACE-Hemmer auch die zunehmende Anzahl von AT1R-Antagonisten (also spezifischen Hemmstoffen am Subtyp 1 des Angiotensin II-Rezeptors, dessen Stimulation durch AngII zur blutdrucksteigernden Wirkung führt), wie zB. Losartan, Valsartan, Candesartan, Eprosartan, Irbesartan, Telmisartan oder Olmesartan, im Bereich der Tumorbehandlung vorgeschlagen.

Trotz ihres (kommerziellen) Erfolges im Bereich der Blutdrucksenkung ist die Verwendung von ACE-Hemmern oder AT1R-Antagonisten aber auch mit Nachteilen verbunden, wie zB. ihre teilweise schweren Nebenwirkungen.

Auch haben sich die auf Basis von ACE-Hemmern oder AT1R-Antagonisten vorgeschlagenen Tumorbehandlungs-Konzepte bislang nicht durchgesetzt, obgleich derartige Vorschläge schon kurz nach Entwicklung der ersten ACE-Hemmer (Captopril 1974; seit 1981 am Markt) bzw. AT1R-Antagonisten (Losartan seit 1996 am Markt) gemacht worden sind. Jedenfalls scheint dieser Mechanismus zwar wissenschaftlich interessant zu sein, eine erfolgreiche praktische Verwendung in der klinischen Tumorbehandlung konnte aber bislang nicht auf breiter Basis realisiert werden.

Ein weiteres essentielles Effektorpeptid des RAS ist Angiotensin 1-7 (Ang 1-7). Dieses Peptid ist der perfekte Antagonist zu Ang II: Während Ang II ein positiver Regulator des RAS ist, kann Ang 1-7 als negativer RAS-Modulator betrachtet werden. Ang 1-7 attenuiert die Effekte von Ang II und weist antihypertensive, anti-inflammatorische, anti-proliferative, antiangiogenetische und vasodilatorische Eigenschaften auf. Es aktiviert die NO-Synthetase und setzt auch die Expression des AT1 Rezeptors herab. Ang 1-7 inhibiert ebenfalls die durch Ang.II induzierte MAP-Kinasen Phosphorylierung. Ang 1-7 verhindert ferner in vitro das Wachstum von Lungenkrebszelllinien und in experimentellen Tumormodellen in der Maus das Wachstum von Tumoren. Interessanterweise wird ACE, das Enzym, welches maßgeblich für die Produktion von Ang II verantwortlich ist, durch Ang 1-7 inhibiert. Ang 1-7 verhindert demzufolge die Synthese seines Antagonisten Ang II. Es wurde auch vorgeschlagen, Ang 1-7 zur Tumorbehandlung einzusetzen, jedoch ist dafür aufgrund der kurzen Halbwertszeit dieses Peptids eine kontinuierliche Infusion erforderlich, welche in der Praxis sehr umständlich und mit großen Einschränkungen für den Patienten verbunden ist.

Interessanterweise existiert ein Schlüsselenzym des RAS, welches das Verhältnis zwischen aktivierendem Ang II und inaktivierendem Ang 1-7 reguliert: Dieses Enzym, ACE2, wurde 1997 entdeckt, jedoch wurde dessen wichtigste Funktion als Modulators des RAS erst im Jahr 2000 erkannt. Als membranverankertes Glykoprotein auf diversen Organen wie Herz, Niere, Leber und Lunge aber auch auf Blutgefäßen wandelt es Ang II zu Ang 1-7 um. Die Expression von ACE2 wird durch diverse Stimuli gesteuert, wobei die zugrunde liegenden Mechanismen bis heute noch nicht vollständig aufgeklärt wurden. Es sind bereits verschiedene weitere Reaktionswege, die unter Mitwirkung/Regulierung von ACE2 stehen, beschrieben. Auch sind möglicherweise viele Reaktionswege neben der Umwandlung von Ang II zu Ang 1-7 noch nicht bekannt. ACE2 wird in Gegenwart inflammatorischer Zytokine hinunterreguliert, was in weiterer Folge zu einer pathologischen Akkumulation von Ang II in den betroffenen Kompartimenten und zu einer Organschädigung führen kann.

Bedingt durch inflammatorische Prozesse in Folge einer Organschädigung oder nach viralen oder bakteriellen Infektionen kommt es zur Ausschüttung inflammatorischer Zytokine, welche die endogene ACE2-Expression und somit die Entstehung von schützendem Ang 1-7 herabsetzen. Reaktives Ang II akkumuliert in weiterer Folge und potenziert den aufkeimenden inflammatorischen Prozess. Ebenso nimmt die Konzentration an reaktiven Sauerstoffspezies im Gewebe zu. In Kombination mit den proliferativen und vaskularisierenden Eigenschaften entsteht ein zunehmend proliferatives Klima, welche die weitere Ang II-Akkumulation fördert, amplifiziert. Um diesem Teufelskreis zu entkommen, hat sich überraschenderweise der Einsatz von ACE2-Aktivität erfindungsgemäß als erfolgreich zur Hemmung des Tumorzellwachstums erwiesen. Somit kann bei einer therapeutische Verabreichung dieser Aktivität erfolgreich eine Ang II-Akkumulation unterbunden oder sogar vorgebeugt und so auch die Inflammation und ein proliferatives Milieu gedämpft werden: Durch eine erhöhte oder wiederhergestellte ACE2-Aktivität werden sofort pathologisch erhöhte Ang II-Konzentrationen abgefangen. Ang 1-7 wird nachgebildet, und schwächt durch dessen anti-inflammatorische Wirkung ebenfalls die Inflammation ab. Weiters limitiert Ang 1-7 durch dessen Eigenschaft, ACE zu inhibieren, die Nachproduktion von Ang II. Ang 1-7 inhibiert die Zellproliferation und senkt in weiterer Folge die Expression von AT1R. Der Einsatz von ACE2-Aktivität ist demzufolge eine effiziente Therapie-Strategie zur Behandlung diverser Tumorerkrankungen, da damit sowohl die zelluläre Entartung, die Neovaskularisierung von heranwachsenden Tumoren und die Metastasierung solider Tumoren unterbunden werden kann.

Durch den erfindungsgemäßen Einsatz der ACE2-Enzymaktivität können durch eine Enzymaktivität, die dem Körper bekannt ist und daher keine Fremdeigenschaft darstellt, die durch die Tumorerkrankung aus dem Gleichgewicht gebrachten molekularen Regelungssysteme wieder in Richtung eines stabilen Ausgangszustand gebracht werden. Im Gegensatz zu den mehr artifiziellen "small molecules", deren Wirkspezifität meist limitiert ist und deren Abbauprodukte Probleme für den Patienten-Metabolismus darstellen können, ist die ACE2-Enzymaktivität in die Gleichgewichts- und Regelungsprozesse des Körpers so eingebunden, wodurch unerwartete Nebenreaktionen sehr unwahrscheinlich sind. Es hat sich auch überraschender Weise gezeigt, dass ACE2 zwar in der Lage ist, die aus dem Gleichgewicht gebrachten Prozesse wieder in Richtung des stabilen Ausgangszustandes zu bringen, eine weitergehende Wirkung über dieses Gleichgewicht hinaus in die andere Richtung jedoch nicht erzielt werden kann, trotz hoher Dosierung der ACE2-Aktivität.

Die formelle Ausnahme von Lungenkrebs aus den erfindungsgemäß zu behandelnden Tumorarten ist auf die WO 2004/000367 zurückzuführen, worin die Verwendung von ACE2 zur Behandlung von Lungenerkrankungen beschrieben ist. Dabei wurden in ACE2-knockout-Mäusen schwere Lungenschäden beobachtet, die in derartigen Mäusen durch ACE2-Gabe verhindert oder reduziert werden konnten. Damit wurde ein Therapiekonzept zur symptomatischen Behandlung derartiger Lungenschäden vor allem beim "Acute Respiratory Distress Syndrome" (ARDS) bestätigt, weil bei ARDS analoge Lungenschäden beobachtet werden. Analoge Lungenschäden waren auch bei anderen Lungenerkrankungen bekannt, so dass in der WO 2004/000367 auch die Verwendung von ACE2-Aktivität für andere Lungenerkrankungen auf Basis des dort geoffenbarten Tiermodells vorgeschlagen worden sind, darunter auch die Behandlung von Lungenkrebs. Es ist jedoch klar, dass die Offenbarung der WO 2004/000367 keinerlei Andeutungen für den Fachmann liefern, die vorgeschlagene Verwendung zur Behandlung von Lungenkrebs auch auf andere Tumorerkrankungen auszuweiten. Die Offenbarung der WO 2004/000367 erschöpft sich für den Fachmann in der Anregung, ACE2-Aktivität zur Behandlung oder Verhinderung konkreter Lungenschäden einzusetzen, die bei verschiedenen Lungenerkrankungen beobachtet werden können. Die WO 2004/000367 enthält aber keine gezielte Offenbarung zur spezifischen und ursächlichen Behandlung von Lungenkrebs bzw. zur Verringerung des Wachstums des Lungentumors oder der entarteten Zellen dieses Tumors. Der Vorschlag in der WO 2004/000367, durch ACE2 eine Verbesserung der Lungenfunktion in Lungenkrebs zu erreichen, bietet daher keinerlei technische Lehre für die vorliegende Erfindung, sondern stellt eben nur eine formale, zufällige punktuelle Überlappung bei Lungenkrebs dar.

Die vorliegende Erfindung ist in einem breiten Spektrum an Tumorerkrankungen anwendbar, im Grunde jedenfalls überall, wo mit dem Tumor über Ang II eine Neovaskularisierung einhergeht, was in allen soliden Tumoren und in vielen hämatopoietischen Krebserkrankungen der Fall ist (zumindest in einigen Stadien derartiger maligner Erkrankungen des Blutes).

Die Internationale statistische Klassifikation der Krankheiten und verwandter Gesundheitsprobleme (ICD-10) klassifiziert maligne Tumoren nach ihrer Lokalisation. Bevorzugte Gruppen erfindungsgemäß zu behandelnder Tumore sind demgemäß ebenfalls derartigen lokalen Gruppen zuzuordnen.

Demgemäß ist die erfindungsgemäß zu behandelnde Tumorerkrankung vorzugsweise ausgewählt aus Tumorerkrankungen des reproduktiven Traktes, insbesondere Eierstockkrebs, Hodenkrebs, Prostatakrebs oder Brustkrebs, Tumorerkrankungen des Verdauungstraktes, insbesondere Magenkrebs, Darmkrebs, Rektalkarzinom, Pankreaskrebs, Speiseröhrenkrebs und Leberkrebs, Nierenkrebs, Melanomen oder Neuroblastomen (wobei hierin die Ausdrücke "Krebs", "Tumor", "Karzinom", etc. immer synonym verwendet werden und sich auf bösartige Erkrankungen beziehen.

Die vorliegende Erfindung eignet sich besonders zur Verhinderung oder Verringerung des Wachstums der Tumorzellen. Diese Wirkung kann vorteilhafter Weise in Kombination mit bekannten Therapie-Konzepten verwendet werden. Vorzugsweise wird die erfindungsgemäße ACE2-Behandlung daher in Kombination mit einer konventionellen Tumortherapie eingesetzt wird, insbesondere in Kombination mit einer Strahlentherapie, einer Chemotherapie, einer Hormontherapie, einer Antikörpertherapie, einer "targeted therapy" wie zB Tyrosinkinasehemmer, und/oder einer operativen Tumorentfernung. Die erfindungsgemäße Behandlung kann auch in einem sehr frühen Stadium der Tumorerkrankungen verwendet werden, wodurch die Heilungschancen erheblich vergrößert werden.

Konventionelle Tumortherapien umfassen die Operation, also die operative Entfernung des Tumors und benachbarter Lymphknoten, die Strahlentherapie (durch radioaktive Stoffe (zB. radioaktives Iod, welches durch die Schilddrüse aktiv aufgenommen wird), durch Röntgenstrahlen, durch Protonentherapie oder Ionenbestrahlung (Bestrahlung mit Protonen oder Ionen, die den Tumor umgebendes Gewebe schont), durch Mikrowellen (Aufheizung des betroffenen Gewebes)), und die Medikamentenbehandlung (durch Zytostatika ("Chemotherapie"; wodurch die Krebszellen an der Vermehrung gehindert bzw. gestoppt werden), durch Hormontherapie (zB. Durch Testosteronentzug beim Prostatakarzinom), durch Blockierung von Wachstumsrezeptor-induzierter Signaltransduktion, durch Hemmung des Blutgefäßwachstums (antiangioneogenentische Mittel) oder durch Immuntherapie (zur Steigerung der Immunantwort gegen Tumorzellen bzw. mit dem Einsatz spezifischer (monoklonaler) Antikörper gegen Tumorantigene oder die Radioimmuntherapie)).

Erfindungsgemäß kann die ACE2-Behandlung auch im Rahmen der palliativen Behandlung bzw. Förderung der Lebensqualität von Tumorpatienten, insbesondere im terminalen Stadium dieser Erkrankungen, zum Einsatz kommen. Die palliative Behandlung kann die Gabe von Schmerzmitteln, die Sicherstellung einer ausreichenden Ernährung, die Hemmung des Knochenabbaues, die Steigerung der Blutbildung im Knochenmark, die symptomatische Behandlungen (zB. Aufdehnung von Stenosen durch Bougierung oder Einlage von Stents) und Physiotherapie derartiger Patienten umfassen.

Besonders bevorzugt wird die ACE2-Aktivität erfindungsgemäß zur Behandlung der Folgen öder Nebenwirkungen der Tumortherapie eingesetzt wird, insbesondere zur Behandlung der Folgen der Strahlentherapie, der Chemotherapie oder der Tumoroperation. Hierbei ist vor allem das Vermögen der ACE2-Aktivität entscheidend, die außer Kontrolle geratenen körpereigenen Regulationssysteme wieder zu "beruhigen".

Aufgrund der bevorzugten systemischen Gabe der ACE2-Aktivität und der Wirkung auf die Verringerung des Wachstums der Tumorzellen eignet sich die vorliegende Erfindung besonders gut zur Verhinderung der Metastasierung der Tumore.

Vorzugsweise werden erfindungsgemäß Tumorerkrankungen behandelt, die durch eine erhöhte Konzentration an Angiotensin II im Tumor, in der Tumorumgebung oder im Tumorpatienten charakterisiert sind. Erhöhte Ang II Konzentrationen können durch ACE2 im Gegensatz zu ACE-Hemmern oder AT1R-Antagonisten nebenwirkungsfrei reduziert werden und so die negativen Wirkungen von Ang II bei der Tumorerkrankung unterbunden werden, sowie simultan die Konzentration des antiproliferativ und anti-inflammatorisch wirkenden Ang 1-7 erhöht werden.

Vorzugsweise werden gemäß der vorliegenden Erfindung maligne Ergüsse, verschiedene Ödeme oder erhöhte vaskuläre Permeabilität im Rahmen der Tumorerkrankungen behandelt.

Wie erwähnt, wird die ACE2-Aktivität vorzugsweise in einer systemisch applizierbaren Form verabreicht, besonders bevorzugt in einer intravenös applizierbaren Form oder in Form eines Nasensprays, insbesondere in liposomaler Form. Jedoch kann ACE2-Aktivität auch in einer lokal applizierbaren Form verabreicht werden, insbesondere in einer intratumoralen oder intradermalen Form. Besonders bevorzugt ist bei der Verabreichung an Patienten die Verwendung einer löslichen Form von ACE2.

"Polypeptid mit ACE2-Aktivität", "ACE2-Polypeptid", "ACE2" oder "ACE2-Aktivität" wird hierin synonym verstanden als eine enzymatische Aktivität, die im chemischen Sinne der Aktivität des natürlichen humanen ACE2 entspricht. Natürliches humanes ACE2 ist eine membranverankerte Carboxypeptidase, die als Rezeptor vor allem auf Lungen-, Nieren- und Herzzellen, aber auch auf Endothelzellen exprimiert wird. ACE2 spaltet diverse Peptidsubstrate, wie Apellin, Bradykinin, aber ebenso Angiotensin-I, welches zu Angiotensin 1-9, aber insbesondere Ang-II, welches zu Ang 1-7 gespalten werden. Ang II und Ang 1-7 sind - wie erwähnt - Antagonisten des RAS. ACE2 ist über die Steuerung der Peptidverhältnisse maßgeblich für die Regulation der Gefäßdicke sowie für die Endothelpermeabilität verantwortlich und beeinflusst hierbei die Homeostase des Organismus. Die Expression von ACE2 ist zytokingesteuert und wird in diversen entzündlichen Erkrankungen herabgesetzt, was in weiterer Folge zu einer pathologischen Anreicherung von Ang II, einem der wichtigsten Substrate von ACE2, führt. Eine "ACE2-Aktivität" gemäß der vorliegenden Erfindung bezieht sich daher auf ein Polypeptid ("ACE2-Polypeptid"), welches jedenfalls in der Lage ist, Ang II spezifisch zu Ang 1-7 umzusetzen.

Besonders bevorzugte ACE2-Polypeptide sind Fragmente des natürlichen humanen ACE2, welche die ACE2-Aktivität aufweisen, dh. Ang II zu Ang 1-7 umzusetzen kann und zwar - im Verhältnis zu natürlichem humanem ACE2 - zu mindestens 10%, vorzugsweise zu mindestens 50 %, noch bevorzugter zu mindestens 100 %, insbesondere zu mindestens 150 %, jeweils bezogen auf die molare Aktivität.

Es hat sich erfindungsgemäß herausgestellt, dass bei Tumoren erhöhtes Ang II durch Erhöhung der ACE2-Aktivität hinunterreguliert werden kann, wodurch auch Ang 1-7 in situ zur Verfügung gestellt wird. Es ist daher mit ACE2 erfindungsgemäß sowohl die positive Wirkung von ACE-Hemmern und AT1R-Antagonisten erzielbar, als auch die positive Wirkung von Ang 1-7 im Patienten nutzbar geworden. Daher eignet sich die Erhöhung der ACE2-Aktivität im Tumorpatienten ausgezeichnet als Therapie diverser Tumorerkrankungen. Exogenes ACE2 kann hierzu zB. als lösliches Protein systemisch verabreicht werden oder dessen endogene Aktivität durch geeignete Aktivatoren oder Agonisten gesteigert werden. Beispiele geeignete ACE2-Aktivatoren oder ACE2-Agonisten sind zB. in der WO 2004/000365 und in der US 6,194,556 B1 beschrieben. Ferner können ebenfalls durch einen geeigneten therapeutischen Ansatz die ACE2 Expression und somit die ACE2 Aktivität erhöht werden. Beispielsweise können durch Einbringen von Nukleinsäuren, die für ein funktionelles ACE2-Enzym (ein ACE2-Polypeptid), kodieren, in den Tumorpatienten erhöhte ACE2-Aktivitäten im Patienten etabliert werden.

Bevorzugt wird erfindungsgemäß aber die Enzymaktivität selbst verabreicht, insbesondere in Form eines rekombinanten ACE2-Produkts. Das erfindungsgemäße ACE2-Produkt ist dabei wie erwähnt vorzugsweise eine lösliche Form des humanen ACE2-Enzyms (welches im Körper in membrangebundener Form vorhanden ist). Das humane Wildtyp (wt) ACE2-Molekül hat 805 Aminosäurereste. Die Aminosäuren 1-17 stellen dabei eine Signalsequenz dar; am C-terminalen Ende ist das Protein hydrophob und ist mit diesem Ende auch in der Membran verankert. Bei der löslichen Form von ACE2 sind daher vorzugsweise die hydrophoben, C-terminalen Bereiche eliminiert, das erfindungsgemäß bevorzugter Weise eingesetzte ACE2-Polypeptid weist daher keine transmembrane Domäne am C-Terminus auf. Bevorzugte Varianten der erfindungsgemäßen ACE2-Aktivität, die zur Tumorbehandlung eingesetzt wird, weisen daher eine Deletion der C-terminalen 60 bis 200 Aminosäuren auf. Besonders bevorzugte Ausführungsformen umfassen dabei lösliche ACE2-Polypeptide, deren Polypeptidkette aus den Aminosäuren 18-740 oder enzymatisch aktiven Fragmenten davon besteht. Ein weiteres bevorzugtes Polypeptid besteht aus den Aminosäuren 18-615 der ACE2-Sequenz oder enzymatisch aktiven Fragmenten davon.

Eine bevorzugte Form der erfindungsgemäßen ACE2-Aktivität ist die dimere Form wie in der EP 08450052.9 beschrieben. Die dimere Form ist - im Gegensatz zur sonst im Stand der Technik beschriebenen monomeren Form - besser löslich in ebenfalls geladenen Lösungen (zB. physiologische Infusionslösungen, Serum, Salzlösungen,...), weist keine Aggregatbildung auf, ist einem reduzierten Proteaseangriff ausgesetzt, weist eine gesteigerte Halbwertszeit auf und ist einfacher zu reinigen.

Der lösliche Abschnitt von ACE2 enthält 7 N-Glykosylierungsstellen. Nicht vollständig glykosyliertes ACE2 ist schlechter löslich, neigt zur Aggregation, ist potentiell immunogen und weist eine verkürzte Halbwertszeit auf. Vorzugsweise ist daher insbesondere das dimere rekombinante ACE2-Polypeptid an mindestens 80% der möglichen N-Glykosylierungspositionen glykosyliert und weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Gemäß der EP 08450052.9 wurde ein Produktionsprozess beschrieben, mit welchem hochreines und aktives vollständig komplex glykosyliertes, dimeres ACE2 reproduzierbar hergestellt werden kann. Dieses Produkt zeichnet sich aufgrund seines hohen Zuckeranteils (>20 Massen %) und der komplexen, stark verzweigten Art der teilweise negativ geladenen Zuckerstrukturen aus. Diese wirken sich positiv auf die Löslichkeit, die Bioverfügbarkeit, die Reinheit, die Aktivität sowie die Pharmakologie des Produktes aus. Durch die Wahl eines geeigneten Expressionskonstruktes, eines geeigneten Expressionswirtes, einer optimierten Selektionsstrategie, durch ein auf den Zellmetabolismus abgestimmtes Medium sowie durch minutiöse begleitende Klonanalytik und Selektion konnte eine Zelllinie hergestellt werden, die zum gewünschten Produkt führt.

Bevorzugt wird erfindungsgemäß ein rekombinantes ACE2 Polypeptid verwendet, welches glykosyliert ist, wobei die Glycogruppen eines ACE2-Polypeptid-Monomers in Summe mindestens 10, 11, 12, 13, 14, 15, oder mindestens 16 Sialylsäurereste aufweisen und das ACE2-Polypetid als Dimer vorliegt. Vorzugsweise enthält das Dimer zwei Zink-Ionen. Unter Sialylsäurereste werden dabei insbesondere Reste vom N-Acetylneuraminsäure-Typ (Neu5Ac) verstanden, im speziellen an N- oder O-Glycosylierungen (wie in der österreichischen Anmeldung A 913/2007 oder der europäischen Anmeldung EP 08450052.9 beschrieben.

Bevorzugte ACE2-Polypeptide weisen demgemäß mind. 70%, vorzugsweise mind. 80%, insbesondere mind. 90%, am meisten bevorzugt 100% der glykosylierten N-Glykosylierungsstellen Sialinsäure auf, vorzugsweise sind die N-Glykosylierungsstellten entsprechend Asn53, Asn90, Asn103, Asn322, Asn432, Asn546, Asn690 der ACE2-Sequenz sialysiert. In speziellen Ausführungen ist ein zu diesen Asn53 Asn90, Asn103, Asn322, Asn432, Asn546 und/oder Asn690 ACE2-Sequenz korrespondierendes Asparagin einzeln oder zusammen einfach, zweifach, dreifach oder vierfach sialylisiert. In einer bevorzugten ACE2-Präparation sind vorzugsweise mindestens 50%, 60%, 70%, 80%, 90%, 95%, 99% oder 100% dieser Aminosäure entweder einfach, zweifach, dreifach oder vierfach sialylisiert.

Vorzugsweise wird erfindungsgemäß eine Präparation von rekombinanten ACE2-Polypeptiden, umfassend ein dimeres ACE2-Polypeptid verwendet, wobei der Anteil von ACE2-Polypeptiden mit einem Molekulargewicht von unter 100 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im Speziellen bei 0%, liegt. Der Anteil wird hierfür z.B. durch native Gelelektrophorese bestimmt. Vorzugsweise liegt der Anteil von ACE2-Polypeptiden mit Transmembrandomänen unter 20%, vorzugsweise unter 10%, insbesondere bevorzugt unter 5%, am meisten bevorzugt unter 1%, im Speziellen bei 0%.

Vorzugsweise liegt der Anteil von ACE2-Multimeren unter 20%, vorzugsweise unter 10%, insbesondere bevorzugt unter 5%, am meisten bevorzugt unter 1%, im Speziellen bei 0%. Unter ACE2-Multimeren werden Komplexe mit 3 oder mehr ACE2-Polypeptiden verstanden. Vorzugsweise beträgt der Anteil an ACE2-Dimeren an ACE2-Molekülen mindestens 10%, 20%, 30%, 40% 50%, 60%, 70%, 80%, 90%, 95% oder mindestens 99%. In weiteren Ausführungsformen kann in Kombination dazu oder unabhängig der Anteil an ACE2-Monomeren an ACE2-Molekülen mindestens 10%, 20%, 30%, 40% 50%, 60%, 70%, 80%, 90%, 95% oder mindestens 99%, sein.

Die erfindungsgemäß zu verwendenden ACE2-Polypeptide weisen vorzugsweise eine katalytische Aktivität des ACE2-Polypeptids oder der Präparation (kkat) von mindestens 4/s, vorzugsweise mindestens 5/s, insbesondere bevorzugt mindesten 6/s, speziell bevorzugt mindestens 7/s, am meisten bevorzugt mindestens 7,6 /s, bezogen auf den Umsatz von Ang II zu Ang 1-7 (Angiotensin 1-7) Umsatz. Der Umsatz kann auf an sich bekannte Art, insbesondere aber wie in den Beispielen der A 913/2000 beschrieben, auf simple Weise getestet werden.

Gemäß einer bevorzugten Ausführungsform der vorliegenden Erfindung werden vor allem Tumor-Patienten mit einer schlechten Prognose für den Patienten erfindungsgemäß mit ACE2-Aktivität behandelt.

Die vorliegende Erfindung wird anhand der nachfolgenden Beispiele, auf die sie selbstverständlich nicht beschränkt ist, nä-. her erläutert.

### BEISPIELE:

### Beispiel 1: Expression von hochglykosyliertem ACE2

Der lösliche Abschnitt der humanen ACE2 Sequenz wurde in einen Expressionsvektor kloniert, in welchen zuvor der amplifizierbare Selektionsmarker DHFR eingefügt wurde, um zu einer erhöhten Expression des ACE2 Genes zu führen. Hierzu wurde zwischen die für ACE2 und DFHR kodierenden Gene eine attenuierte IRES eingebracht, welche die bi-cistronische Ablesung von ACE2 und DHFR auf derselben mRNA ermöglicht. Nachdem beide Proteine unter Kontrolle desselben Promotors exprimiert werden, kann über DHFR Selektion unter Verwendung des Antagonisten MTX zielstrebig die ACE2 Expression gesteigert werden. Durch diese Strategie ist es möglich, besonders stabile Expressionszelllinien zu erhalten, die hohe Ausbeuten eines Produktes konstanter Qualität liefern. Dies ermöglicht es auch in Zelllinien vernünftige Produkttiter zu erzielen, die möglicherweise für die rekombinante Expression eines bestimmten Targetproteins minder geeignet sind.

Dieser Vektor wurde in CHOdhfr- transfiziert und unter kontinuierlich gesteigertem MTX Druck wurde die Kopienanzahl der ACE2 Gene amplifiziert. Über mehrere Selektions- und Subklonierungsrunden wurden die besten Produzenten mittels intrazellulärer FACS Analytik und proteinchemischer sowie enzymatischer Analysen auf optimale Produkteigenschaften selektiert: Es wurden für die Auswahl des bestgeeigneten Klons vor allem die spezifische enzymatische Aktivität, welche mit 3 verschiedenen Substraten gemessen wurde, die Produkthomogenität, die zelluläre Produktivität jedoch auch die Zuckerkomplexizität in Betracht gezogen. Die Produkteigenschaften wurden durch spezifische Selektion hochglykosylierender Klone verbessert, um enzymatisch hoch aktives und komplex N-glykosyliertes ACE2 zu exprimieren.

Während lösliches ACE2 ein Molekulargewicht von 83 kDa aufweist, werden Klone ausgewählt, welche im SDS-PAGE, bedingt durch deren Zuckerstruktur, im Bereich von bis zu 120 kDa erschienen. Die präliminären Klone wurden im Weiteren auf proteinfreies Wachstumsmedium umgestellt. Dieses Medium ist chemisch definiert und auf die rekombinante Expression von Glykoproteinen in CHO abgestimmt worden. Alle Klone wurden in Kultur gehalten und auf deren Produktionsprozesstauglichkeit überprüft. Im Speziellen wurden Wachstumsraten aufgezeichnet sowie die Überstände auf Produktverlauf und Metaboliten untersucht. Wiederum wurden die Expressionsprodukte sowie die Klone genau analysiert.

Alle Klone exprimierten hochaktives ACE2 und wiesen Produktivitäten um 20-30 pg/Zelle/Tag auf. Weiters wurden ebenfalls die Zuckerstrukturen und deren Heterogenität analysiert. Es wurden Klone ausgewählt, bei denen in ACE2 alle 7 N-Glykosylierungsstellen prozessiert waren, wobei diese mindestens eine bi-, manche jedoch auch eine tri-antennäre komplexe Glykosylierung mit terminalen Sialinsäureh aufwiesen. Auf der Basis des letztlich selektierten Klones wurde eine Mastercellbank hergestellt und getestet, sowie ein GMP tauglicher Reinigungs- und in weiterer Folge ein GMP Produktionsprozess aufgebaut.

Das gemäß diesem Beispiel hergestellt rACE2 wird als Dimer erhalten. Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren.

Diese Dimerisierung eines vollständig N-Glykosylierten ACE2 wurde durch Expression in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Atome werden in den Komplex eingebaut und stabilisieren dessen Struktur. Die starke Ladung der Zuckerketten solvatisiert das Molekül in physiologischen wässrigen Lösungen und zwingt die zugehörigen geladenen Proteindomänen nach außen. Der Produktionsprozess wurde so aufgebaut, dass im Endprodukt ausschließlich ACE2 Dimere vorkommen. Dies wird dadurch ermöglicht, dass bei Generierung des rACE2 genügend Zn²⁺-Ionen vorhanden sind (vorzugsweise kommen 1,5 - 5 micromolar Zn²⁺ zur Anwendung, insbesondere kann die Fermentation bei 2,5-3,5 uM Zn²⁺ durch geführt werden) und anschließend die weiteren Behandlungsschritte in Anwesenheit von Zn²⁺-Ionen vorgenommen werden.

### Beispiel 2: Pharmakologische Produkteigenschaften

Die gemäß Beispiel 1 hergestellt Dimer-ACE2 Präparation liegt als stabile, hochreine und konzentrierte Proteinlösung in physiologischer Pufferung vor und kann ohne weitere Stabilisierung gelagert und verabreicht werden.

Dieses ACE2 zeigt aufgrund des hohen Zuckeranteils keine Aggregation zu Multimeren. Ferner weist die ACE2 Präparation die volle enzymatische Aktivität auf.

ACE2 kann aufgrund seiner Löslichkeit als Bolus i.v., aber auch subkutan verabreicht werden. Die Bioverfügbarkeit ist aus denselben Gründen systemisch sofort nach Gabe gewährleistet.

Aufgrund des hohen, stark verzweigten und komplexen Zuckeranteils wird ACE2 nur langsam abgebaut. Es resultiert daraus eine lange terminale Halbwertszeit von mindestens 10.5 Stunden, welche in diversen Spezies, unter anderem auch in Rhesus-Makaken gemessen wurde.

Der hohe Sialinsäureanteil bedingt ferner, dass gegen ACE2 im Menschen keine neutralisierende Immunantwort aufgebaut wird. Diese wäre nicht nur für die exogene Gabe von ACE2 kontraproduktiv, sondern könnte auch autologes, zellständiges ACE2 neutralisieren. Die beschriebene ACE2-Formulierung mitsamt sämtlicher einhergehender Produkteigenschaften ermöglicht daher erst eine effiziente Therapie mit rhACE2.

### Beispiel 3: Bestimmung der spezifischen ACE2 Aktivität

Die spezifische Aktivität von ACE2 Präparationen wurde durch Messung des Umsatzes von Ang II (Asp-Arg-Val-Tyr-Ile-His-Pro-Phe) bestimmt. Sämtliche Messungen wurden als Dreifachbestimmungen in einem Ansatzvolumen von 100 µl durchgeführt. Die enzymatische Reaktion wurde durch Zugabe von 250 ng/ml ACE2 zu einer 80 µM Ang II Lösung in 50 mM MES, 300 mM NaCl, 10µM ZnCl2 und 0,01% Brij-30 bei pH 6,5 gestartet. Die Proben wurden sorgfältig gemischt und für genau 18 Minuten bei 37°C inkubiert. Die enzymatische Reaktion wurde durch Zugabe von 100 mM EDTA gestoppt. Zur Analyse wurden die Lösungen mittels RP-HPLC (Waters C18 µBondapak, 2,1x300mm, 10µm, 125Å) unter Verwendung eines linearen Gradienten von 10 bis 60% CH3CN in 0,08% H3PO4 über 20 Minuten bei einer Flussrate von 1 ml/min aufgetrennt. Ferner wurden sowohl Ang II als auch Ang 1-7 Peaks in den Chromatogrammen erkannt und integriert. Die Peptidkonzentrationen wurden anhand von Kalibrationskurven ermittelt. Ferner wurde der enzymatische Umsatz sowie die spezifische Enzymaktivität bestimmt.

Die gemäß Beispiel 1 hergestellte ACE2-Präparation weist eine katalytische Aktivität kkat von 8,0±0,3 /s gemessen anhand des Ang II Umsatzes und 8,8±0,2 /s in Bezug auf den Ang 1-7 Umsatz auf. Beide Werte stimmen gut überein und sind deutlich höher als die Angaben von Vickers et al. (J Biol Chem. 2002 277(17):14838-43), der eine katalytische ACE2 Aktivität von 3,5 /s veröffentlicht hat. Die Reaktionbedingungen waren identisch.

Die Ursache der 240% höheren Aktivität gemäß der vorliegenden Präparation dürften post-translationelle Modifikationen und hier vor allem die N-Glykosylierung sein, die in dem Material, das Vickers verwendet hat, deutlich geringer ausgeprägt war. Das dort beschriebene Material wurde in Insektenzellen exprimiert und wies zwar dieselbe Aminosäuresequenz auf, war jedoch zu einem deutlich geringeren Anteil und Verzweigungsgrad glykosyliert. Es wurde ferner auch eine im Handel erhältliche ACE2-Präparation von R&D systems (cat. 933-ZN), die ebenfalls eine deutlich geringere Aktivität kkat von 2,0±0,1 /s aufwies, untersucht. Eine wesentliche Eigenschaft der erfindungsgemäß bevorzugt zu verwendenden Dimer-Präparation ist also die erstaunlich hohe Aktivität, die vor allem durch post-translationelle Modifikationen ermöglicht wird.

### Beipiel 4: Unterdrückung des Tumorzell-Wachstums

Eine humane Tumorzelllinie wurde in einer Zelldichte von 2,5x10⁴ Zellen/ml in 200 µl RPMI 1640 in 10% FCS in 96 Wellplatten ausgesät und bei 37°C und 5% CO₂ inkubiert. Der Einfluss diverser aktiver Komponenten des RAS wurde anhand der folgenden Versuchsansätze evaluiert. Sämtliche Analysen wurden als Dreifachbestimmungen durchgeführt (sACE2 = lösliches ACE2 ohne C-terminale Membrandomäne):
Bedingung A. Kulturmedium RPMI 1640 mit 10% FCS als Kontrolle
Bedingung B. Kulturmedium supplementiert mit 100 nM Ang II
Bedingung C. Kulturmedium supplementiert mit 100 nM Ang 1-7
Bedingung D. Kulturmedium supplementiert mit 20 µg/ml sACE2
Bedingung E. Kulturmedium supplementiert mit 20 µg/ml sACE2 und 100 mM Ang II

Täglich wurden 100 µl Medium abgezogen und durch dasselbe Volumen an frischem, spezifischem Medium ersetzt. Die Zellzahl wurde an den Tagen 2, 3, 6, 8, 10, 13, 15 und 17 mittels Mehrfachzählung im Hämazytometer bestimmt. Für jede Bestimmung wurde eine separat angesetzte Testplatte verwendet.

Signifikante Unterschiede in der Zellzahlbestimmung bedingt durch unterschiedliche Wachstumsbedingungen wurden ab dem vierten Kulturtag sichtbar. Der Zusatz von Ang II bewirkte ein deutlich erhöhtes Zellwachstum. Ang 1-7 hingegen reduzierte das Zellwachstum. Während ACE2 alleine (mangels Substrat verständlicher Weise) ein marginal erhöhtes Wachstum induzierte, senkte die Zugabe von ACE2 in Gegenwart von Ang II das Zellwachstum, ähnlich wie durch den Zusatz von Ang 1-7 alleine. ACE2 neutralisierte demzufolge nicht nur das durch Ang II induzierte gesteigerte Zellwachstum, sondern inhibierte dieses über Effekte des Peptides Ang 1-7. Dieses wurde offensichtlich nur gebildet, wenn neben ACE2 auch Ang II dem Wachstumsmedium zugesetzt wurde.

Demgemäß konnte auch mit diesem Beispiel gezeigt werden, dass ACE2 die "aktivierende" Ang II Schiene dämpfen und die "attenuierende" Ang 1-7 Seite aufbauen kann. Beide Effekte bewirken, wie experimentell belegt, ein verlangsamtes Tumorzellwachstum.

## Patentansprüche

1. : Verwendung eines Polypeptids mit einer Angiotensin konvertierenden Enzym 2 (angiotensin converting enzyme 2; ACE2)-Aktivität zur Herstellung eines Medikaments zur Behandlung von Tumorerkrankungen, ausgenommen Lungenkrebs.

2. : Verwendung nach Anspruch 1, **dadurch gekennzeichnet, dass** die Tumorerkrankung ausgewählt ist aus Tumorerkrankungen des reproduktiven Traktes, insbesondere Eierstockkrebs, Hodenkrebs, Prostatakrebs oder Brustkrebs, Tumorerkrankungen des Verdauungstraktes, insbesondere Magenkrebs, Darmkrebs, Rektalkarzinom, Pankreaskrebs, Speiseröhrenkrebs und Leberkrebs, Nierenkrebs, Melanomen oder Neuroblastomen.

3. : Verwendung nach Anspruch 1 oder 2, **dadurch gekennzeichnet, dass** ACE2 zur Behandlung der Folgen oder Nebenwirkungen der Tumortherapie eingesetzt wird, insbesondere zur Behandlung der Folgen der Strahlentherapie, der Chemotherapie oder der Tumoroperation.

4. : Verwendung nach einem der Ansprüche 1 bis 3, **dadurch gekennzeichnet, dass** ein Polypeptid mit ACE2-Aktivität zur Verhinderung der Metastasierung der Tumore eingesetzt wird.

5. : Verwendung nach einem der Ansprüche 1 bis 4, **dadurch gekennzeichnet, dass** die Tumorerkrankungen durch eine erhöhte Konzentration an Angiotensin II im Tumor, in der Tumorumgebung oder im Tumorpatienten charakterisiert sind.

6. : Verwendung nach einem der Ansprüche 1 bis 5, **dadurch gekennzeichnet, dass** das Polypeptid mit ACE2-Aktivität zur Behandlung von malignen Ergüssen, Ödemen oder von erhöhter vaskulärer Permeabilität im Rahmen der Tumorerkrankungen eingesetzt wird.

7. : Verwendung nach einem der Ansprüche 1 bis 6, **dadurch gekennzeichnet, dass** das Polypeptid mit ACE2-Aktivität in einer systemisch applizierbaren Form vorliegt, vorzugsweise in einer intravenös applizierbaren Form oder in Form eines Nasensprays, insbesondere in liposomaler Form.

8. : Verwendung nach einem der Ansprüche 1 bis 6, **dadurch gekennzeichnet, dass** das Polypeptid mit ACE2-Aktivität in einer lokal applizierbaren Form vorliegt, insbesondere in einer intratumoralen, subkutanen oder intradermalen Form.

9. : Verwendung nach einem der Ansprüche 1 bis 8, **dadurch gekennzeichnet, dass** eine lösliche Form von ACE2 verwendet wird.

10. : Verwendung nach einem der Ansprüche 1 bis 9, **dadurch gekennzeichnet, dass** eine dimere Form von ACE2 verwendet wird.

11. : Verwendung nach einem der Ansprüche 1 bis 10, **dadurch gekennzeichnet, dass** das Polypeptid mit ACE2-Aktivität in Kombination mit einer konventionellen Tumortherapie eingesetzt wird, insbesondere in Kombination mit einer Strahlentherapie, einer Chemotherapie, einer Antikörpertherapie und/oder einer operativen Tumorentfernung.

12. : Verwendung nach einem der Ansprüche 1 bis 11, **dadurch gekennzeichnet, dass** die Tumorerkrankung durch eine schlechte Prognose für den Patienten charakterisiert ist.

13. : Polypeptid mit einer Angiotensin konvertierten Enzym 2 (angiotensin converting enzyme 2; ACE2)-Aktivität zur Anwendung in der Behandlung von Tumorerkrankungen, ausgenommen Lungenkrebs.

14. : Polypeptid zur Anwendung nach Anspruch 13, weiters wie nach einem der Ansprüche 2 bis 12 definiert.

## Claims

1. Use of a polypeptide with an angiotensin converting enzyme 2 (ACE2) activity for the production of a medicament for the treatment of tumor diseases other than lung cancer.

2. The use according to claim 1, **characterized in that** the tumor disease is selected from tumor diseases of the reproductive tract, in particular ovarian cancer, testicular cancer, prostate cancer or breast cancer, tumor diseases of the digestive tract, in particular stomach cancer, intestinal cancer, rectal carcinoma, pancreatic cancer, esophageal cancer and liver cancer, kidney cancer, melanomas or neuroblastomas.

3. The use according to claim 1 or 2, **characterized in that** ACE2 is used for the treatment of the effects or side effects of the tumor therapy, in particular for the treatment of radiotherapy, chemotherapy or tumor-surgery effects.

4. The use according to any one of claims 1 to 3, **characterized in that** a polypeptide with ACE2 activity is used for the prevention of tumor metastasis.

5. The use according to any one of claims 1 to 4, **characterized in that** said tumor diseases are **characterized by** an increased angiotensin-II concentration in the tumor, in the tumor environment or in the tumor patient.

6. The use according to any one of claims 1 to 5, **characterized in that** the polypeptide with ACE2 activity is used for the treatment of malignant effusions, edemas or increased vascular permeability associated with said tumor diseases.

7. The use according to any one of claims 1 to 6, **characterized in that** the polypeptide with ACE2 activity is present in a systemically applicable form, preferably in an intravenously applicable form or in the form of a nasal spray, in particular in a liposomal form.

8. The use according to any one of claims 1 to 6, **characterized in that** the polypeptide with ACE2 activity is present in a locally applicable form, in particular in an intratumoral, subcutaneous or intradermal form.

9. The use according to any one of claims 1 to 8, **characterized in that** a soluble ACE2 form is used.

10. The use according to any one of claims 1 to 9, **characterized in that** a dimeric ACE2 form is used.

11. The use according to any one of claims 1 to 10, **characterized in that** the polypeptide with ACE2 activity is used in combination with conventional tumor therapy, in particular in combination with radiotherapy, chemotherapy, antibody therapy and/or surgical tumor removal.

12. The use according to any one of claims 1 to 11, **characterized in that** said tumor disease is **characterized by** a poor prognosis for the patient.

13. A polypeptide with an angiotensin converting enzyme 2 (ACE2) activity for use in the treatment of tumor diseases other than lung cancer.

14. A polypeptide for use according to claim 13, further being defined according to any of the claims 2 to 12.

## Revendications

1. Utilisation d'un polypeptide présentant une activité de l'enzyme de conversion de l'angiotensine 2 (angiotensin converting enzyme 2 ; ACE) pour la production d'un médicament destiné au traitement de maladies tumorales à l'exception du cancer du poumon.

2. Utilisation selon la revendication 1, **caractérisée en ce que** la maladie tumorale est choisie parmi les maladies tumorales du système reproducteur, en particulier le cancer des ovaires, le cancer des testicules, le cancer de la prostate ou le cancer du sein, les maladies tumorales des voies digestives, en particulier, le cancer de l'estomac, le cancer de l'intestin, le cancer du rectum, le cancer du pancréas, le cancer de l'oesophage et le cancer du foie, le cancer des reins, les mélanomes ou neuroblastomes.

3. Utilisation selon la revendication 1 ou 2, **caractérisée en ce que** l'ACE2 est utilisée pour le traitement des suites ou des effets secondaires de la thérapie tumorale, en particulier pour le traitement des suites de la radiothérapie, de la chimiothérapie ou de l'opération d'une tumeur.

4. Utilisation selon l'une des revendications 1 à 3, **caractérisée en ce qu'**un polypeptide présentant une activité de l'ACE2 est utilisé pour empêcher le développement métastasique des tumeurs.

5. Utilisation selon l'une des revendications 1 à 4, **caractérisée en ce que** les maladies tumorales sont **caractérisées par** une concentration accrue en angiotensine II dans la tumeur, dans l'environnement de la tumeur ou chez le patient présentant la tumeur.

6. Utilisation selon l'une des revendications 1 à 5, **caractérisée en ce que** le polypeptide présentant une activité d'ACE2 est utilisé pour le traitement d'épanchements malins, d'oedèmes ou de perméabilité vasculaire accrue dans le cadre des maladies tumorales.

7. Utilisation selon l'une des revendications 1 à 6, **caractérisée en ce que** le polypeptide présentant une activité de l'ACE2 se présente sous une forme applicable au niveau systémique, de préférence sous une forme applicable par voie intraveineuse ou sous une forme de spray nasal, en particulier, sous une forme liposomale.

8. Utilisation selon l'une des revendications 1 à 6, **caractérisée en ce que** le polypeptide présentant une activité de l'ACE2 se présente sous une forme applicable localement, en particulier sous une forme intra-tumorale, sous-cutanée ou intradermique.

9. Utilisation selon l'une des revendications 1 à 8, **caractérisée en ce qu'**une forme soluble de l'ACE2 est utilisée.

10. Utilisation selon l'une des revendications 1 à 9, **caractérisée en ce que** l'on utilise une forme dimère de l'ACE2.

11. Utilisation selon l'une des revendications 1 à 10, **caractérisée en ce que** le polypeptide présentant une activité de l'ACE2 est utilisé en combinaison avec une thérapie tumorale classique, en particulier, en combinaison avec une radiothérapie, une chimiothérapie, une thérapie par anticorps et/ou une résection tumorale chirurgicale.

12. Utilisation selon l'une des revendications 1 à 11, **caractérisée en ce que** la maladie tumorale est **caractérisée par** un pronostic défavorable pour le patient.

13. Polypeptide présentant une activité de l'enzyme de conversion de l'angiotensine 2 (angiotensin converting enzyme 2 ; ACE) pour l'utilisation dans le traitement de maladies tumorales à l'exception du cancer du poumon.

14. Polypeptide pour l'utilisation selon la revendication 13, tel que défini en outre selon l'une des revendications 2 à 12.
